# EUROPÄISCHE PATENTANMELDUNG

(11) **EP 1 046 372 A1**
(43) Veröffentlichungstag der Anmeldung: **25.10.2000**
(21) Anmeldenummer: 99107961.7
(22) Anmeldetag: 22.04.1999
(51) Int. Cl.: A61B 5/0416, H01R 4/24

(54) **Elektrische Schneidkontaktierung, insbesondere für medizinische Einmalartikel wie fetale Skalp-Elektroden**

(71) Anmelder: Hewlett-Packard Company, Palo Alto, California 94304 (US)
(72) Erfinder: Schmid, Alfons, 71034 Böblingen (DE)
(74) Vertreter: Barth, Daniel Mathias

(57) **Zusammenfassung**

Beschrieben wird ein erstes Kontaktierungsteil (20) zur Herstellung eines elektrischen Kontakts mit einem zweiten Kontaktierungsteil (30) indem beim Zueinanderführen der beiden Kontaktierungsteile (20, 30) ein Kontaktierungselement (50) in einen elektrischen Leiter (60, 63) einschneidet. Dabei weist das erste Kontaktierungsteil eine Umformung (20) auf, die den elektrischen Leiter (60, 63) kontaktierungsseitig elektrisch isolierend umgibt.

## Beschreibung

Die vorliegende Erfindung betrifft Kontaktierungsteile zur Herstellung eines elektrischen Kontakts indem beim Zueinanderführen der Kontaktierungsteile ein Kontaktierungselement in einen elektrischen Leiter einschneidet.

Steckverbindungen sind im allgemeinen lösbare oder nicht lösbare elektrische Verbindungen eines oder mehrerer Kontakte, wobei ein elektrischer Kontakt durch Ineinanderstecken komplementärer Kontaktteile, wie Stecker und Buchse, bewirkt wird.

Bei lösbaren Kontaktierungen erfolgt eine Verbindung regelmäßig derart, daß sich ein Kontaktteil federnd an das andere legt oder mit dem anderen verklemmt wird. Hierbei gibt es sehr viele Variationen, wie zum Beispiel Buchsen und federnde Stifte, federnde Buchsen und Stifte, Plattfedern auf Flächen, Scherklemmen, Klemmen von Drähten, Klemmen von Stiften, Schraubklemmen von Drähten oder Stiften und so weiter. Derartige Steckverbindungen werden üblicherweise für häufige Steckzyklen vorgesehen.

Im Gegensatz zu den lösbaren elektrischen Verbindungen soll bei den nicht lösbaren elektrischen Verbindungen eine elektrische Kontaktierung hergestellt werden, die regelmäßig nur eine einmalige, nicht lösbare elektrische Verbindung darstellt. Zu den nicht lösbaren elektrischen Verbindungen zählen insbesondere die Schneid-Klemm-Verbindungen, bei denen einmalig ein Kontakt durch ein isoliertes Kabel mit Hilfe eines Schneidelements hergestellt wird. Die Kontaktierung erfolgt hierbei normalerweise senkrecht zum Mantel der elektrischen Leitungen.

Neben den im strengen Wortsinn zu verstehenden lösbaren oder nicht lösbaren elektrischen Verbindungen sind schließlich noch Mischformen anzuführen, die für mehr oder minder häufige Steckzyklen ausgelegt sind, also beispielsweise solche Schneid-Klemm-Verbindungen, die für eine bestimmte Anzahl von Steckzyklen vorgesehen werden.

Ein typisches Beispiel für eine lösbare elektrische Verbindung wird derzeit im medizinischen Bereich der Geburtsüberwachung für die sogenannten fetalen Kopfschwarten- oder Skalp-Elektroden zur Überwachung der fetalen Herztätigkeit verwendet. Mit der fetalen Skalp-Elektrode wird das EKG vom ungeborenen Kind mittels zweier Elektroden abgenommen. Auf der Skalp-Elektroden-Seite stehen dabei zwei offene (abisolierte), verzinnte Drähte, die für einen Kontakt mit zwei federnden Backen als Gegenkontakte auf der Geräteseite vorgesehen sind. Derartige fetalen Skalp-Elektroden werden von der Anmelderin unter anderem beschrieben in US-A-5,423,314 oder US-A-3,750,650.

Die fetale Skalp-Elektrode ist gewöhnlicherweise ein Einmalprodukt und sollte daher, wie andere medizinische Einmalprodukte ebenso, möglichst preisgünstig sein. Die einfachen blanken Kontaktdrähte erfüllen dabei diese Kostenbedingung.

Internationale Vorschriften wie FDA (Food and Drug Administration) oder MDD (Medical Device Directive) fordern, daß bei den fetalen Skalp-Elektroden die offenen, elektrisch leitenden Drähte durch einen isolierten Steckverbinder ersetzt werden müssen, um die Patienten gegen Fehlkontaktierungen zu schützen.

Eine weitere Anforderung gerade bei fetalen Skalp-Elektroden ist weiter, daß das Steckerteil möglichst klein ist, da es bei den bekannten Ausführungsformen durch ein Innenrohr eines Einführungsinstrumentes leicht durchziehbar sein muß.

Eine derartige Steckverbindung für fetale Skalp-Elektroden, die insbesondere die genannten Sicherheitsvorschriften einhält, ist aus EP-A-484107 bekannt. Bei diesem Stecksystem werden (entsprechend der bekannten Klinkensteckerverbindung) die beiden Drähte mit isolierten Buchsen verbunden, die dann auf Pins aufgesteckt werden. Nachteilig an dieser Anordnung ist jedoch, daß aufgrund der geforderten Größenlimitation des Steckerteils dieses nur sehr aufwendig und damit kostenintensiv hergestellt werden kann.

### ZUSAMMENFASSUNG DER ERFINDUNG

Es ist deshalb Aufgabe der vorliegenden Erfindung eine elektrische Kontaktierungsweise zur Verfügung zu stellen, die es erlaubt, daß einerseits isolierte Steckverbinder verwendet werden können, zum Beispiel zur Erfüllung der genannten Sicherheitsvorschriften, und andererseits, daß Steckerteile möglichst klein dimensionierbar sind. Auch sollte die erfindungsgemäße Kontaktierungsweise es erlauben möglichst preisgünstige Steckkontakte zu schaffen, die sich insbesondere für medizinische Einmalprodukte, wie zum Beispiel fetale Skalp-Elektroden, eignen. Die Aufgabe wird gelöst durch die Merkmale der unabhängigen Ansprüche. Vorteilhafte Ausführungsformen sind in den abhängigen Ansprüchen angeführt.

Die erfindungsgemäße Lösung eignet sich insbesondere dazu, die für fetale Skalp-Elektroden notwendigen Sicherheitskontaktierungen zu ermöglichen. Allerdings ist die erfindungsgemäße Lösung nicht auf medizinische Anwendung im allgemeinen oder fetale Skalp-Elektroden im speziellen beschränkt, sondern eignet sich für alle lösbaren Steckverbindungen, bei denen für die Steckerseite nur eine geringere Steckzyklenzahl notwendig ist. Dies trifft insbesondere für Einmalprodukte oder sonstige Anwendungen zu, bei denen eine lösbare Kontaktierung geschaffen werden soll, diese jedoch regelmäßig nur ein oder wenige Male geschlossen oder geöffnet wird.

Bei Verwendung der erfindungsgemäßen Lösung, insbesondere bei fetalen Skalp-Elektroden, wird sichergestellt, daß die Sicherheitsvorschriften bei geringen Kosten eingehalten werden können. Außerdem wird der elektrische Verbindungsvorgang für den Anwender wesentlich vereinfacht. Da der Benutzer normalerweise Schutzhandschuhe trägt und diese durch das vaginale Einführen der fetalen Skalp-Elektrode feucht sind, ist das Bedienen der herkömmlich üblichen Federbacken und das Einlegen der abisolierten Drähte nicht einfach.

Dies wird durch eine erfindungsgemäße Steckverbindung wesentlich verbessert, da der Anwender auch mit einer Hand den Stecker sicher bedienen kann.

Unabhängig von dem jeweiligen Einsatzgebiet erlaubt es die erfindungsgemäße Lösung Steckkontakte, die für geringere Steckzyklenzahlen benötigt werden, zu geringen Kosten zu schaffen. Insbesondere erlaubt es die Erfindung auch nur einen Teil der Steckerverbindung, vorzugsweise das isolierte Stekkerteil, sehr preisgünstig herstellen zu können. Das zweite Teil der Steckverbindung ist dabei zu Kosten herzustellen, die den momentan eingesetzten Lösungen entsprechen (so entspricht beispielsweise der Aufwand des Verzinnens der abisolierten Drähte einem Umspritzen der Kabel mit einem erfindungsgemäßen Stecker). Das bedeutet, daß der Vorteil des erfindungsgemäßen Lösungsprinzips insbesondere in den Kosten für solche Steckverbindungen liegen kann, bei denen beispielsweise der Stecker preisgünstig sein sollte und nur für eine geringe Steckzyklenzahl benötigt wird. Somit eignet sich die Erfindung insbesondere für Einmalartikel, wie sie gerade im medizinischen Bereich aus hygienischen Gründen häufig anzutreffen sind.

Eine bevorzugte Ausführungsform einer erfindungsgemäßen Steckverbindung weist auf der einen Seite, der Kabelseite, ein auf ein (vorzugsweise nicht abisoliertes) Kabel sitzendes Steckerteil, daß vorzugsweise mit Kunststoff auf das Kabel überspritzt ist, und auf der anderen zu kontaktierenden Seite ein Buchsenteil zur Aufnahme des Steckerteils auf. Insbesondere bei Verwendung des gleichen Kunstsstoffmaterials für das Steckerteil wie für die Kabelisolation kann eine gute Verbindung zwischen Steckerteil und Kabelisolation erreicht werden. Das Steckerteil auf der Kabelseite ist dabei so geformt, daß es vorzugsweise verdrehgesichert in die Öffnung des Buchsenteils eingeschoben werden kann. Während des Einschiebevorgangs schneidet eine in einen offenen Raum des Buchsenteils hineinragende Kontaktschneide durch die Isolation in den Kabelleiter und erzeugt damit eine elektrische Verbindung zwischen einem Kontaktteil des Buchsenteils und dem Kabelleiter. Entsprechend können bei einer Vielzahl von Kabelleitern eine Vielzahl von Kontaktschneiden vorgesehen werden, die jeweils eine elektrische Verbindung zwischen einem entsprechenden Kontaktteil des Buchsenteils und dem jeweiligen Kabelleiter herstellen.

In einer bevorzugten Ausführungsform ist das Buchsenteil als entnehmbares bzw. auswechselbares Teil vorgesehen, um so ein schnelles Auswechseln der Buchse, z.B. im Falle einer nachlassenden Schneidintensität oder für ein Reinigen oder Sterilisieren des Buchsenteils, zu gewährleisten. Je nach Anwendung kann das Buchsenteil auch als Einmalartikel ausgeführt sein.

In einer anderen Ausführungsform kann das Buchsenteil steckbar mit einem Verbindungselement, wie z.B. mit einem Meßgerät oder einer Beinplatte (bei der fetalen Skalp-Elektrode), verbunden werden, um so ein schnelles und einfaches Auswechseln zu gewährleisten. Dabei kann durch entsprechende Auslegung und/oder Dimensionierung dieser Steckverbindung eine evtl. vorgegebene Auszugkraft erreicht werden, bei der sich das Buchsenteil unter Zugeinwirkung aus der Verbindung mit dem Verbindungselement löst. Dies kann insbesondere bei medizinischen Anwendungen wie der fetalen Skalp-Elektrode erforderlich oder erwünscht sein.

### KURZE BESCHREIBUNG DER ZEICHNUNGEN

Die Erfindung wird im folgenden weiter unter Heranziehung der Zeichnungen erläutert, wobei sich gleiche Referenzzeichen auf funktional gleiche oder ähnliche Merkmale beziehen.
- Figur 1: zeigt eine bevorzugte Ausführungsform einer erfindungsgemäßen elektrischen Steckverbindung 10,
- Figur 2: zeigt in größerem Detail und in mehreren Ansichten das in Figur 1 gezeigte Steckerteil 20,
- Figuren 3A und 3B: zeigen in größerem Detail die in Figur 1 dargestellte Buchse 30,
- Figuren 4A und 4B: zeigen alternative Ausführungsformen von Schneiden 50,
- Figur 5: zeigt eine geeignete Ausformung einer Schneide 50, und
- Figur 6: zeigt eine Ausführungsform des Buchsenteils 30.

### DETAILLIERTERE BESCHREIBUNG DER ZEICHNUNGEN

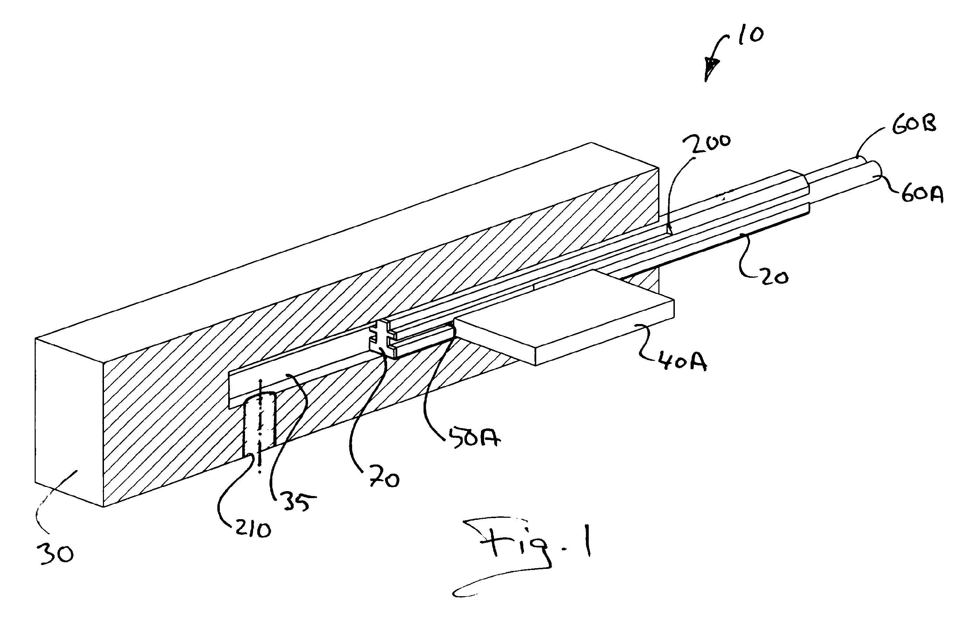

Figur 1 zeigt eine bevorzugte Ausführungsform einer erfindungsgemäßen elektrischen Steckverbindung 10. Ein Steckerteil 20 (im Folgenden auch nur Stecker genannt) ist dabei teilweise in ein aufgeschnitten dargestelltes Buchsenteil 30 (im Folgenden auch nur Buchse genannt) eingeführt. Die Formen von Stecker 20 und Buchse 30 sind aufeinander angepaßt, wobei die Begriffe Stecker und Buchse hier so verwendet werden sollen, daß das Stekkerteil in einen Buchsenkanal 35 des Buchsenteils einführbar (oder einsteckbar) ist. Ein mit der Buchse 30 verbundenes Kontaktteil 40A trägt an seiner dem Steckerteil 20 zugewandten Seite eine (in Figur 1 nur teilweise sichtbare) (Kontakt-)Schneide 50A. Die Schneide 50A soll im kontaktierten Zustand einen elektrischen Kontakt zwischen dem (vorzugsweise nach außen zu führenden) Kontakt 40A der Buchse 30 und zumindest einem entsprechenden elektrischen Leiter des Steckers 20 herstellen.

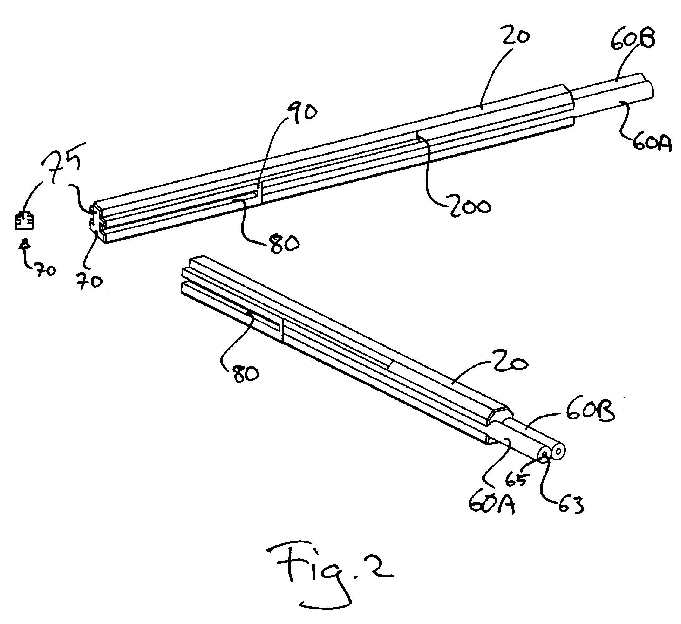

In dem in Figur 1 gezeigtem Ausführungsbeispiel weist der Stecker 20 zwei Kabel (oder Drähte) 60A und 60B auf, die jeweils ummantelte und damit isolierte elektrische Leiter (vgl. 63 in Fig. 2) enthalten. Die Schneide 50A des Kontaktes 40A soll einen elektrischen Kontakt zu dem Leiter 60A herstellen und ein weiterer Kontakt 40B mit einer entsprechenden Schneide 50B (in der Figur 1 gewählten Darstellungsweise jeweils nicht sichtbar) ist zur Kontaktierung des Leiters 60B vorgesehen.

Das Steckerteil 20 wird vorzugsweise durch Überspritzen, beispielsweise mit einem Kunststoff, der isolierten Kabel 60 hergestellt, was eine kostengünstige Fertigung des Steckerteils 20 erlaubt. Anstelle der in Figur 1 gezeigten zwei isolierten Drähte 60A und 60B können je nach Anwendung ein oder mehrere Drähte 60 verwendet werden.

Figur 2 zeigt in größerem Detail und in mehreren Ansichten das in Figur 1 gezeigte Steckerteil 20. Die äußere Ausformung des Steckerteils 20 ist im wesentlichen komplementär zu der äußeren Ausformung der Buchse 30, um so ein Ineinanderstecken von Stecker 20 und Buchse 30 zu gewährleisten.

In der in den Figuren 1 und 2 gezeigten bevorzugten Ausführungsform weist das Steckerteil 20 vorzugsweise weiter eine geeignete Außenformung auf, um so das Steckerteil 20 in seiner Positionierung gegenüber dem Buchsenteil 30 zu sichern und ein ungewünschtes Verdrehen auszuschließen. Auf diese Weise kann bei mehreren Kabeln 60 beispielsweise ein polaritätsrichtiges Kontaktieren der einzelnen Kabel oder bzw. daß die einzelnen Kabel 60 den jeweils entsprechenden Kontakten 40 zugeordnet werden sichergestellt werden. In dem in den Figuren 1 und 2 gezeigten Beispiel weist das Steckerteil 20 an seiner der Buchse 30 zugewandten Stirnseite 70 in etwa die Form zweier spiegelbildlich aneinander gerückter und auf dem Kopf stehender F's oder, anders ausgedrückt, eines auf dem Kopf stehenden T's mit einer Mittelstrebe parallel zu der Kopfhorizontalen auf. Ein sich somit ergebendes, zu einer entsprechenden Mittelhorizontalen H (in Einführungsrichtung) nicht achsensymmetrisches (und in Fig. 2 sich oberhalb der oberen Horizontalen befindliches) Kopfteil oder Steg 75 gewährleistet ein orientierungsrichtiges Einfügen des Steckers 20 in die Buchse 30.

Durch geeignete Formgebung des Steckerteils 20 (insbesondere entsprechend der Stirnseite 70) lassen sich ferner auch Kodierungen unterschiedlicher Stekkertypen erreichen, um so beispielsweise ein ungewünschtes Anschließen an eine nicht geeignete Buchse 30 zu verhindern.

Für den Fall, daß eine Verdrehsicherung des Steckerteils 20 nicht erforderlich ist, kann das Steckerteil 20 und insbesondere die Stirnseite 70 auch entsprechend (achsen- oder punkt-) symmetrisch aufgebaut sein, so daß ein Kontaktieren des Steckerteils 20 mit der Buchse 30 in mehreren Drehwinkeln, beispielsweise jeweils 180°, ermöglicht wird. In diesem Falle wäre beispielsweise eine H - Form (z.B. auf der Seite liegend wie in Fig. 2 gezeigt) oder dergleichen geeignet.

Die geeignet ausgeführte Formgebung des Steckerteils 20 (vorzugsweise durch Ummantelung oder Überspritzen der Kabel 60) umfaßt steckerseitig die isolierten Kabel 60, legt deren Position fest und gibt ihnen Halt. Auf der Seite des Steckerteils 20, auf der die Kontaktschneide 50 in den jeweiligen Draht 60 eingreift, befindet sich vorzugsweise eine Aussparung oder Ausnehmung 80, die verhindern soll, daß das oder die Messer 50 unnötig vor der eigentlichen Kontaktierung belastet werden.

Um eine hinreichende Isolation der Kabelenden der Kabel 60 in Steckerrichtung sicherzustellen, werden diese Kabelenden vorzugsweise nicht bis zu der Stirnseite 70, sondern nur bis zu einem vorgebbaren Abstand dazu ausgeführt. Um die Isolation weiter zu erhöhen, ist in einer bevorzugten Ausführungsform, vorzugsweise zwischen dem Ende des jeweiligen Kabels 60 und dieser Ausnehmung 80, eine dünne Schicht 90, vorzugsweise aus einem Kunststoff, vorgesehen.

Wie aus dem unteren Teil der Fig. 2 ersichtlich, sind die Kabel 60 vorzugsweise jeweils aus einem (elektrischen) Leiter 63 und einer den Leiter 63 umgebenden Isolation 65 aufgebaut. Der Leiter 63 kann dabei ein Einzelleiter sein oder aus einer Vielzahl von einzelnen Leiterfasern oder Litzen bestehen. Allerdings ist die Erfindung nicht auf die Kontaktierung derartiger in Fig. 2 gezeigter Kabel beschränkt, sondern es können beliebige andere Kabel verwendet werden. Insbesondere muß sich das Steckerteil 30 nicht an die Isolation 65 anschließen, sondern kann auch direkt an dem Leiter 63 anliegen, so daß beispielsweise auch solche Kabel verwendet werden können, bei denen steckerseitig die Isolation 65 entfernt wurde.

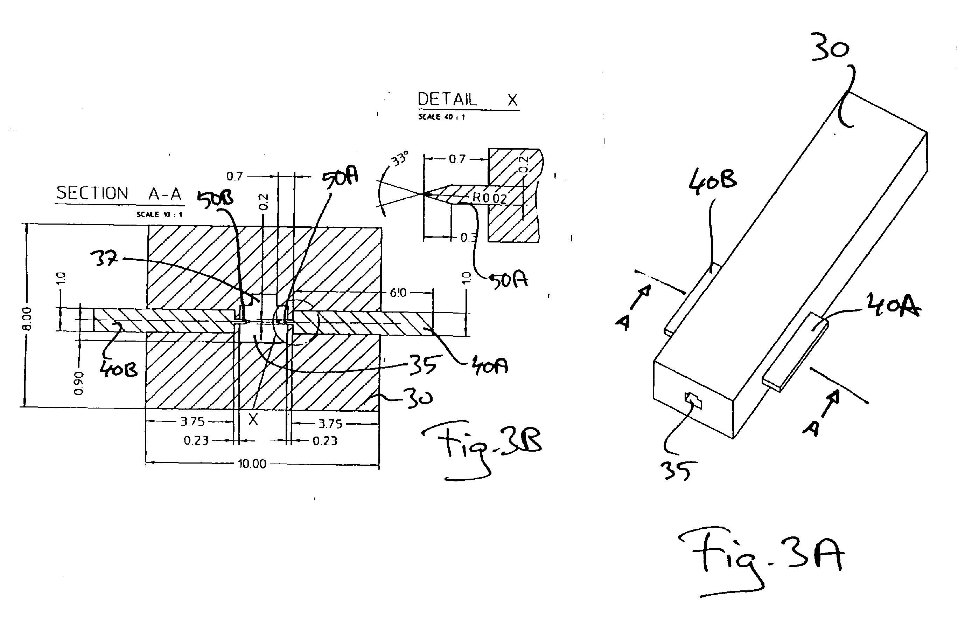

Die Figuren 3A und 3B zeigen in größerem Detail die in Figur 1 dargestellte Buchse 30, wobei Figur 3A eine perspektivische Darstellung der Buchse 30 und Figur 3B eine Schnittbilddarstellung an der Stelle A-A und in Sichtrichtung der Pfeile A zeigt. Die Kontakte 40A und 40B stehen sich an entgegengesetzten Seiten des Buchsenkanals 35 gegenüber, wobei jeweils die Schneiden 50A und 50B in den Buchsenkanal 35 hineinreichen.

Die Formgebung des Buchsenkanals 35 korrespondiert im wesentlichen mit der Formgebung des Steckers 20, um ein leichtes Ineinanderfügen zu gewährleisten. Gleichzeitig muß die Formgebung des Buchsenkanals 35 auf eine gegebenenfalls ausgeführte Verdrehsicherung und/oder Kodierung des Steckers 20 ausgerichtet sein und weist dementsprechende Verdrehsicherungs- und/oder Dekodierungselemente auf. In dem Ausführungsbeispiel der Figuren 3A und 3B weist der Buchsenkanal 35 entsprechend der Formgebung des in Fig. 2 gezeigten Steckers 20 eine zu dem Steg 75 komplementäre Ausnehmung 37 auf.

In den Figuren 3A und 3B ist das den Schneiden 50 entgegengesetzte Ende der Kontakte 40 zur externen Kontaktierung aus dem Gehäuse der Buchse 30 herausgeführt. Allerdings sind auch beliebige andere Herausführungen oder dergleichen der Kontakte 40 aus der Buchse 30 entsprechend der jeweiligen Anwendung möglich. So können beispielsweise die Kontakte 40 in der Buchse 30 in Richtung der Kabelachse innerhalb der Buchse 30 durchgeführt und an einer der Steckereinführungsöffnung des Buchsenkanals 35 entgegengesetzten Seite der Buchse 30 herausgeführt werden.

Durch Einschieben (vgl. Figur 1) des Steckers 20 (vgl. Figur 2) in das Buchsenteil 30 (vgl. Figuren 3B) wird die Kontaktierung erzeugt. Die (Kontakt-) Schneiden 50 werden vorzugsweise als Messer 50 ausgeführt und sind derart angeordnet, daß sie während des Einführungsvorgangs zunächst in der Aussparung oder Schlitz 80 (vgl. Figur 2) des Steckers 20 gleiten, um dann (aus Sicherheitsgründen, um Berührungsschutz zu gewährleisten) nach einer gewissen Tiefe in die isolierten Kabel 60 einzuschneiden und (elektrischen) Kontakt zu erzeugen.

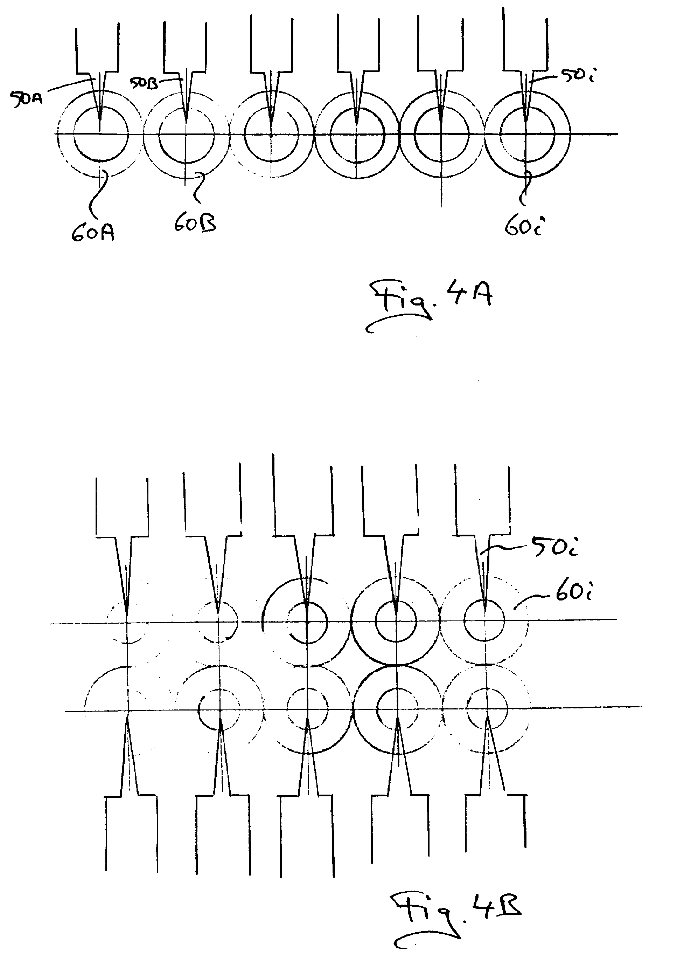

Die Figuren 4A und 4B zeigen alternative Ausführungsformen von Schneiden 50 für die Kontaktierungen mit Kabeln 60 mit einer Vielzahl von Einzelkabeln 60i (mit i = A, B, ...). In Figur 4A sind die Kabel in einer Reihe parallel zueinander angeordnet. Entsprechend weist die Buchse 30 eine Vielzahl von Kontaktschneiden 50i aneinandergereiht in einer Reihe auf. In Figur 4B hingegen weist der Stecker 20 zwei zueinander parallele Reihen von Kabeln 60i auf, und entsprechend weist die Buchse 30 jeweils eine Reihe von Kontaktschneiden 50i oberhalb und unterhalb der Kabelreihen 60i auf.

In einer bevorzugten Ausführungsform wird für die Isolation der Kabel 60i das gleiche Material, vorzugsweise ein Kunststoff, wie für das Steckerteil 20 verwendet, wobei bei Verwendung eines Kunststoffs vorzugsweise das Steckerteil 20 mit diesem Kunststoff über die Kabel 60i überspritzt wird. Durch die entsprechende Auswahl der Materialien für die Ummantelung/Isolation der Kabel 60i und des Steckerteils 20 und vorzugsweise dadurch, daß gleiche Materialien verwendet werden, kann eine enge Verbindung dieser Materialien gewährleistet werden.

In einer bevorzugten Ausführungsform sind die Kontaktschneiden oder-Messer 50i aus einem Material hergestellt, das sehr hart und rostfrei ist und einen guten Kontakt gewährleistet. Ferner sind die Kontaktmesser 50i derart geformt, daß sie so lange wie möglich scharf bleiben, um eine genügend große Anzahl von Steckzyklen zu gewährleisten.

Durch das Vorsehen der Aussparungen 80 und die damit verbundene exakte, genügend lange Führung des Steckerteils 20 im Buchsenkanal 35 des Buchsenteils 30 wird sichergestellt, daß auf die Schneiden 50i der Kontakte 40i keine Querkräfte kommen und somit ein Bruch der Schneiden 50i vermieden werden kann. Bei der Schneide 50i selbst muß unterschieden werden zwischen dem schneidenden Teil und dem Teil, der den Kontakt herbeiführt. Der schneidende Teil sollte möglichst lang sein, um immer einem scharfen Anteil zu haben, während der Teil, der den Kontakt herstellt, möglichst schräg, zum Beispiel in einem Winkel kleiner als 45°, zu eventuell im Kabel 60i enthaltenen Litzen stehen sollte, um zu vermeiden, daß alle Fasern (oder Litzen) zur gleichen Zeit aufgespalten werden müssen.

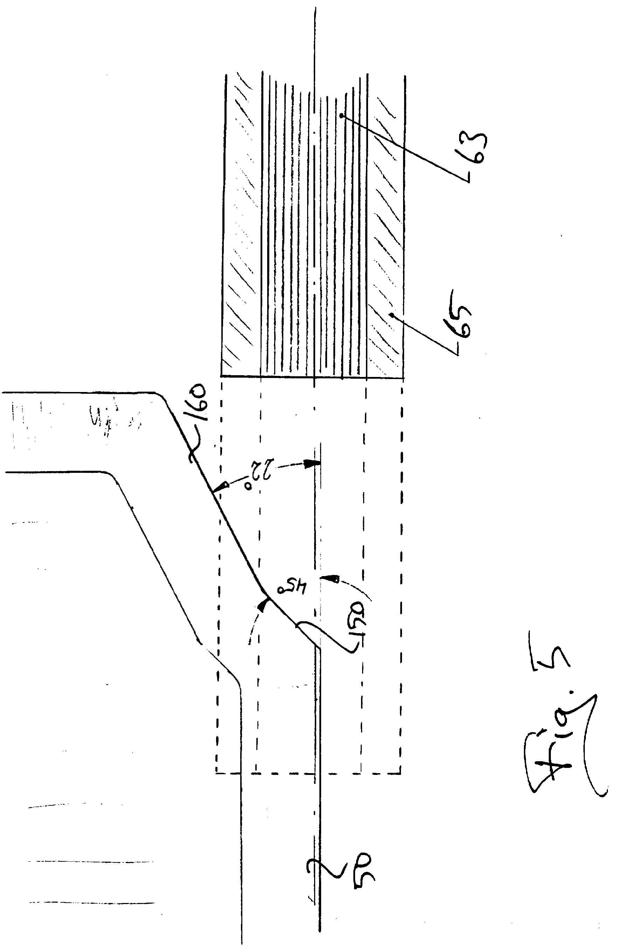

Figur 5 zeigt eine geeignete Ausformung einer Schneide 50. Die Schneide weist einen ersten, vorzugsweise um etwa 40-50° (vorzugsweise 45°) abgewinkelten Bereich für die Kontaktbildung mit dem Leiter 63 des Kabels 60 auf. An den ersten Bereich 150 schließt sich ein zweiter Bereich 160 an, der vorzugsweise um etwa 10-30° (vorzugsweise 22°) abgewinkelt ist und dazu dient, die Isolation 65 und einen kleinen Teil der Leiter 63 anzuschneiden. In Versuchen konnte gezeigt werden, daß mit einer derartigen Ausformung der Schneide 600 Steckzyklen ohne weiteres möglich sind.

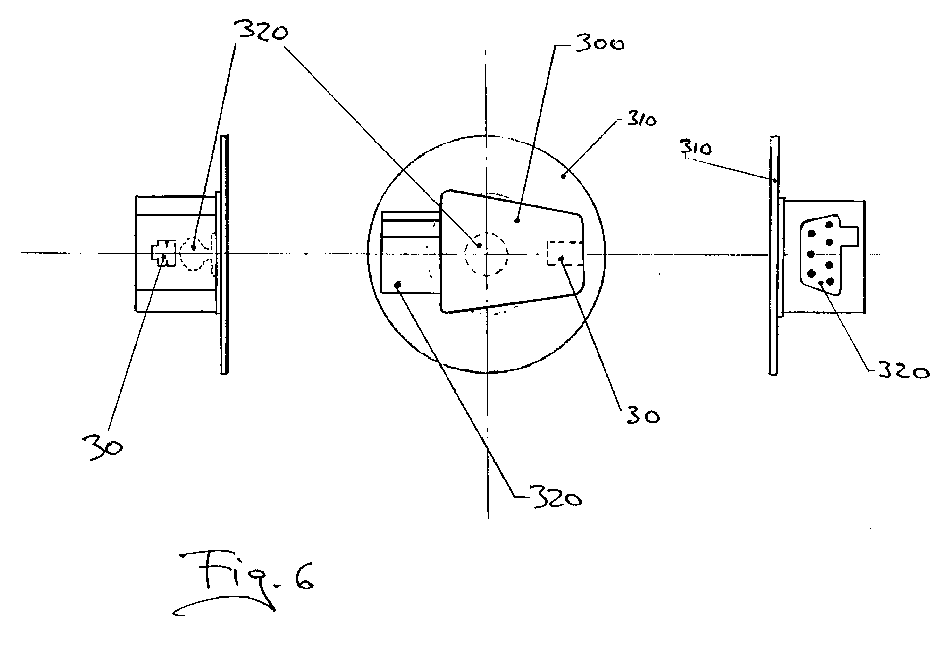

Fig. 6 zeigt in Ansichten von oben und den Seiten eine bevorzugte Ausführungsform des Buchsenteils 30, die erlaubt, daß der Stecker 20 sich bei Zug entgegengesetzt des Buchsenteils 30 aus diesem löst. Zudem ermöglicht diese Lösung, daß das Buchsenteil 30 leicht ausgewechselt werden kann, z.B. im Falle nachlassender Schneidintensität oder zum Reinigen oder Sterilisieren des Buchsenteils 30. Ein Kunststoffkörper 300, der auf der einen Seite die erfindungsgemäße Schneidbuchse 30 trägt, wird auf einen Trägerkörper 310 (wie z.B. eine Standard-EKG-Klebeelektrode) mit Hilfe einer Druckknopfverbindung 320 aufgeschnappt. Im Falle der Standard-EKG-Klebeelektrode 320 bildet diese zusammen mit dem Kunststoffkörper 300 beispielsweise eine passive Beinplatte zur Befestigung am Bein der Patientin. Vorzugsweise trägt der Kunststoffkörper 300 auf der anderen Seite eine weitere Kontaktverbindung 330, wie z.B. eine Buchse gemäß US-A-5,615,674.

Bei Verbindung von Stecker 20 und Buchse 30 und bei Zugbeanspruchung z.B. am Kabel 60 (nicht gezeigt in Fig. 6) dreht sich der Kunststoffkörper 300 um die Druckknopfverbindung 320 in die Zugrichtung. Dadurch greift der Zug immer in der Auszugsrichtung des Steckers 20 an und ermöglicht so ein leichtes Öffnen der Steckverbindung.

Der Stecker 20 wird vorzugsweise durch die mit dem Schneidvorgang bewirkten Reibungskräfte in der Buchse 30 gehalten. Allerdings kann neben oder an Stelle dieser Klemmverbindung auch eine andere Halterung, z.B. durch ein Verriegeln und/oder Festsetzen (beispielsweise durch Zapfen oder Pins), vorgesehen werden.

Ein bevorzugtes Ausführungsbeispiel eines erfindungsgemäßen Kontaktierungssystems soll nun exemplarisch für die Kontaktierung einer fetalen Skalp-Elektrode dargestellt werden. Dabei soll insbesondere auf die Figuren 1-3 und 5 Bezug genommen werden, wobei die genannten Figuren nicht auf die Verwendung für die fetale Skalp-Elektrode beschränkt sind.

Das Steckerteil 20 (als das preisgünstigere Teil) zeigt rechts und links in der Darstellung in Figur 2 die Ausnehmungen 80 für die Schneiden 50, die auf der Buchsenseite in den Figuren 3 dargestellt sind. In dem Bereich der Ausnehmungen 80 sind die Schneiden 50 frei und nicht im Eingriff mit dem Stecker 20. Beim weiteren Einführungsvorgang des Steckers 20 in die Buchse 30 (über den Bereich der Ausnehmungen 80 hinausgehend) wird dann die vorzugsweise sehr kurze Isolationsschicht 90 durchschnitten, um dann die mit der Isolierung 65 versehenen Leiter 63 der Kabel 60 aufzuschneiden und bei weiterem Einschieben des Steckerteils 20 den Kontakt zu den Kupferlitzen des Leiters 63 herzustellen.

Als Verdrehsicherung und zur Verbesserung der Führung zwischen Buchse 30 und Stecker 20 ist am oberen Bereich des Steckers 20 der Steg 75 und am oberen Bereich des Buchsenkanals 35 die zu dem Steg 75 komplementäre Ausnehmung 37 vorgesehen, die sicherstellt, daß der Stecker 20 nur in einer Ausrichtungsweise in die Buchse 30 eingeführt werden kann.

Ein in den Figuren 1 und 2 gezeigter Anschlag 200 des Steckers 20 ist dafür vorgesehen den Einführvorgang zu stoppen. Dabei stößt der Anschlag 200 des Steckers 20 an die dem Stecker zugewandte Frontfläche der Buchse 30 an. Dieser Stop 20 ist insbesondere bei der fetalen Anwendungsweise sinnvoll, da bei einer Verschmutzung des Buchsenkanals 35 dieser Schmutz in den hinteren Bereich des Buchsenkanals 35 geschoben und dort durch eine in Figur 1 gezeigte Öffnung 210 nach außen verdrängt werden kann. Dies wäre bei einem Anschlag an der Endfläche des Buchsenkanals 35 nicht gesichert.

Zur Herstellung des Steckerteils 20 werden die in Figur 1 gezeigten Kabel 60A und 60B in ein entsprechendes Werkzeug eingelegt und rechts und links in Position gehalten. Dann erfolgt ein Kunststoffspritzvorgang, der eine Verbindung zwischen Kunststoffkörper und der Isolation 65 der Kabel 60 herbeiführt.

Dadurch wird der Stecker 20 mechanisch stabil und kann gut in die Buchse 30 eingeschoben werden. Eine Kabeltülle oder Knickschutz kann vorgesehen werden, ist jedoch für die Anwendung der fetalen Skalp-Elektrode nicht erforderlich, da der hier gewünschte Stecker 20 vorzugsweise sehr klein und duktil ist.

Die für die Kontaktierung des Steckerteils 20 mit der fetalen Skalp-Elektrode vorgesehene Buchse 30 ist entsprechend der Figuren 3A und 3B aufgebaut, kann jedoch auch andere Ausführungsformen aufweisen. Die Schneiden 50, die mit den Kontakten 40 fest verbunden sind, werden vorzugsweise in der richtigen Position fest in die Buchse 30 eingespritzt. Die Schneiden 50 selbst haben vorzugsweise die in Figur 5 beschriebene Form.

Die Buchse 30 wiederum wird vorzugsweise entsprechend Fig. 6 zusammen mit einer Standard-Fetal-Buchse gemäß US-A-5,615,674 in einem Kunststoffkörper 300 auf einer Standard-EKG-Klebeelektrode 310 mit Hilfe einer Druckknopfverbindung 320 aufgeschnappt. Die Standard-EKG-Klebeelektrode 320 bildet zusammen mit dem Kunststoffkörper 300 eine passive Beinplatte zur Befestigung am Bein der Patientin.

## Patentansprüche

1. Ein erstes Kontaktierungsteil (20) zur Herstellung eines elektrischen Kontakts mit einem zweiten Kontaktierungsteil (30) indem beim Zueinanderführen der beiden Kontaktierungsteile (20, 30) ein Kontaktierungselement (50) in einen elektrischen Leiter (60, 63) einschneidet, gekennzeichnet durch:
eine Umformung (20), die den elektrischen Leiter (60, 63) kontaktierungsseitig elektrisch isolierend umgibt.

2. Das erste Kontaktierungsteil (20) nach Anspruch 1, dadurch gekennzeichnet, daß die Umformung (20) einen Ausnehmungsbereich (80) aufweist, wobei der Ausnehmungsbereich (80) zur Führung des Kontaktierungselements (50) während einer ersten Phase des Zueinanderführens der beiden Kontaktierungsteile (20, 30) dient und/oder der Ausnehmungsbereich (80) derart geformt ist, daß während dieser ersten Phase das Kontaktierungselement (50) nicht oder nur geringfügig in den elektrischen Leiter (60, 63) einschneidet.

3. Das erste Kontaktierungsteil (20) nach Anspruch 1 oder 2, gekennzeichnet durch eine Verdrehsicherung (75) zur Sicherung eines positionsgenauen Zueinanderführens der beiden Kontaktierungsteile (20, 30) und/oder eine Kodierung zur Sicherstellung, daß erste Kontaktierungsteil (20) nur bestimmten vorgebbaren Typen der zweiten Kontaktierungsteile (30) einen elektrischen Kontakt herstellen kann.

4. Das erste Kontaktierungsteil (20) nach Anspruch 3, dadurch gekennzeichnet, daß die Umformung (20) zur Herstellung der Verdrehsicherung (75) und/oder der Kodierung einen Querschnitt aufweist, der zumindest zu einer Mittelachse nicht achsensymmetrisch ist.

5. Das erste Kontaktierungsteil (20) nach einem der Ansprüche 1-4, dadurch gekennzeichnet, daß die Umformung (20) einen Anschlag (200) zur Verhinderung des Zueinanderführens der beiden Kontaktierungsteile (20, 30) über einen vorgebbaren Punkt hinaus aufweist.

6. Das erste Kontaktierungsteil (20) nach einem der Ansprüche 2-4, soweit auf Anspruch 2 bezogen, dadurch gekennzeichnet, daß die Umformung (20) zwischen dem kontaktierungsseitigen Ende des elektrischen Leiters (60, 63) und dem Ausnehmungsbereich (80) eine dünne Schicht (90), vorzugsweise aus einem Kunststoff, aufweist.

7. Ein zweites Kontaktierungsteil (30), gekennzeichnet durch:
einen Kontaktierungskanal (35) zur Aufnahme des ersten Kontaktierungsteils (20) nach einem der Ansprüche 1-7, und
ein frei in den Kontaktierungskanal (35) hineinragendes Kontaktierungselement (50) zum Einschneiden in einen elektrischen Leiter (60, 63) des ersten Kontaktierungsteils (20) während des Zueinanderführens der beiden Kontaktierungsteile (20, 30) zur Herstellung eines elektrischen Kontakts zwischen den beiden Kontaktierungsteilen (20, 30).

8. Das zweite Kontaktierungsteil (30) nach Anspruch 7, dadurch gekennzeichnet, daß das Kontaktierungselement (50) aufweist:
einen ersten, gegenüber einer Einführungsachse abgewinkelten Bereich (150), vorzugsweise um etwa 40-50°, zur Kontaktbildung mit einem elektrisch leitfähigen Bereich (63) des elektrischen Leiters (60, 63), und
ein zweiten, gegenüber der Einführungsachse abgewinkelten Bereich (160), vorzugsweise um etwa 10-30°, der sich an den ersten Bereich (150) anschließt, zum Anschneiden einer den elektrisch leitfähigen Bereich (63) des elektrischen Leiters (60, 63) umhüllenden Isolation (65) und eventuell eines kleinen Teils des elektrisch leitfähigen Bereichs (63),
wobei beim Zueinanderführen der beiden Kontaktierungsteile (20, 30) zunächst der zweite Bereich (160) des Kontaktierungselements (50) mit dem elektrischen Leiter (60, 63) in Kontakt tritt, und der Winkel des zweiten Bereichs (160) größer ist als der Winkel des ersten Bereichs (150).

9. Das zweite Kontaktierungsteil (30) nach Anspruch 7 oder 8, dadurch gekennzeichnet, daß der Kontaktierungskanal (35) in einem Endbereich in Einführungsrichtung eine Öffnung zum Auslaß von sich im Kontaktierungskanal (35) angesammelten Materialien, wie Verunreinigungen, aufweist.

10. Das zweite Kontaktierungsteil (30) nach einem der Ansprüche 7-9, dadurch gekennzeichnet, daß das zweite Kontaktierungsteil (30) Mittel zum lösbaren Ankoppeln an ein weiteres Teil aufweist, so daß das zweite Kontaktierungsteil (30) als auswechselbares Element an das weitere Teil ankoppelbar ist, um so insbesondere im Falle einer nachlassenden Schneidintensität des Kontaktierungselements (50) oder für ein Reinigen oder Sterilisieren des zweiten Kontaktierungsteils (30) ein Auswechseln des zweiten Kontaktierungsteils (30) zu gewährleisten.

11. Das zweite Kontaktierungsteil (30) nach Anspruch 10, dadurch gekennzeichnet, daß das zweite Kontaktierungsteil (30) steckbar an das weitere Teil ankoppelbar ist, wobei vorzugsweise eine Auszugkraft vorgebbar ist, bei der sich das zweite Kontaktierungsteil (30) unter Zugeinwirkung aus der Verbindung löst.

12. Ein medizinischer Einwegartikel, vorzugsweise eine fetale Skalp-Elektrode, mit einem Kontaktierungsteil (20, 30) nach einem der Ansprüche 1-11.
